# EUROPEAN PATENT APPLICATION

(11) **EP 4 275 717 A1**
(43) Date of publication of application: **15.11.2023**
(21) Application number: 21917770.6
(22) Date of filing: 28.12.2021
(51) Int. Cl.: A61M 5/155, A61M 5/303, C12M 1/00, C12N 15/87, C12N 15/89

(54) **INJECTOR**

(30) Priority: 05.01.2021 JP 2021000519
(71) Applicant: Daicel Corporation, Osaka 530-0011 (JP)
(72) Inventor: SAKAGUCHI, Yuko, Tokyo 108-8230 (JP); TERAI, Kazuhiro, Tokyo 108-8230 (JP)
(74) Representative: Grünecker Patent- und Rechtsanwälte PartG mbB
(86) International application number: PCT/JP2021/048866
(87) International publication number: WO 2022/149550

(57) **Abstract**

An object of the present disclosure is to provide an injector in which at least when a solution containing a biomolecule is injected into an injection target, a rate of the biomolecule that functions in the injection target is large. The object is achieved by an injector configured to inject a solution containing a biomolecule and a gas that is predetermined into an injection target without using an injection needle, the injector including a storage portion configured to store the solution containing the biomolecule and the gas, a nozzle portion communicating with the storage portion, the nozzle portion including an ejection port configured to eject the solution containing the biomolecule and the gas toward the injection target, and a pressurization portion configured to pressurize the solution containing the biomolecule and the gas that are stored in the storage portion during an operation and to eject the solution containing the biomolecule and the gas from the ejection port toward the injection target.

## Description

### TECHNICAL FIELD

The present disclosure relates to an injector.

### BACKGROUND ART

Examples of an injector that injects a medical liquid into a living body or the like include not only a needle-containing injector that performs injection through an injection needle and a needleless injector that performs injection without using an injection needle, but also a catheter provided with an injection needle and a drive source that transport the medical liquid to an injection target.

Among these injectors, the needleless injector may employ a configuration of ejecting an injection component by applying a pressure to a storage chamber that stores an injection solution due to pressurized gas, a spring, or electromagnetic force. For example, a configuration is adopted in which a plurality of nozzle holes are formed inside an injector body, and pistons that are driven in ejection are disposed in correspondence with the respective nozzle holes (Patent Literature 1). With this configuration, the injection solution is simultaneously ejected from the plurality of nozzle holes and uniform injection to a target is intended to be achieved. Then, a plasmid containing a luciferase gene is injected into a rat, which allows cell transfer with high efficiency.

In addition, there is a form in which pressurized gas is used as a power source for ejecting an injection solution in a needleless injector. For example, a pressurization form is exemplified in which large pressurization is instantaneously performed at an initial stage of injection, and then the pressurization force is gradually reduced over 40 to 50 msec (Patent Literature 2).

On the other hand, a method of injecting not only a medical liquid but also air in a mixed state during injection is known. For example, it has been reported that when azacitidine, which is a therapeutic agent for myelodysplastic syndrome, is subcutaneously administered with a needle-containing injector, normal administration without mixing air causes side effects that make a patient uncomfortable, such as reddening of an injection mark, pain at an injection area, or bruising at an injection area, whereas administration with air mixed prevents contact between the epithelium and the medical liquid and reduces the side effects (Non-Patent Literature 1).

In addition, in a case of injection using a needleless injector, when a medical liquid is injected in a state where the medical liquid and the skin of a patient are in contact with each other in advance at the ejection port of a medical liquid chamber, impact force generated by collision of a drive bar with the medical liquid is greatly attenuated due to a time length sensed between the medical liquid and the skin of the patient. Thus, a technique is disclosed in which a gas pocket is provided on the ejection port side in the medical liquid chamber and then the medical liquid is injected (Patent Literature 3).

Furthermore, an in vitro test has been reported in which DNA can be efficiently introduced into a cell of an adherent established cell line by containing air in a storage chamber of a needleless injector (Patent Literature 4), although a target of the test is not a living body.

### PRIOR ART DOCUMENTS

### PATENT LITERATURE

Patent Literature 1: JP 2004 358234 A
Patent Literature 2: US 2005/010168 A1
Patent Literature 3: JP 2002 143302 A
Patent Literature 4: WO 2020/116353 A1

### NON-PATENT LITERATURE

Non-Patent Literature 1: Can. Oncol. Nurs. J., 22(4): 222-34, 2012

### SUMMARY OF THE INVENTION

### PROBLEMS TO BE SOLVED BY THE INVENTION

An object of the present disclosure is at least to provide an injector in which when a solution containing a biomolecule is injected into an injection target, a rate of the biomolecule that functions in the injection target is high.

### MEANS FOR SOLVING THE PROBLEMS

One embodiment of the present disclosure is
an injector configured to inject a solution containing a biomolecule and a gas that is predetermined into an injection target without using an injection needle, the injector including
a storage portion configured to store the solution containing the biomolecule and the gas,
a nozzle portion communicating with the storage portion, the nozzle portion including an ejection port configured to eject the solution containing the biomolecule and the gas toward the injection target, and
a pressurization portion configured to pressurize the solution containing the biomolecule and the gas that are stored in the storage portion during an operation and to eject the solution containing the biomolecule and the gas from the ejection port toward the injection target.

In a preferred aspect of the injector, a volume of the gas stored in the storage portion is equal to or greater than 20% and equal to or less than 60% of a capacity of the storage portion.

In a preferred aspect of the injector, the gas is air.

In a preferred aspect of the injector, the biomolecule is DNA containing a gene.

One embodiment of the present disclosure can also provide a method of injecting a solution containing a biomolecule and a gas that is predetermined into an injection target by using said needleless injector.

### EFFECT OF THE INVENTION

The present disclosure can achieve at least an effect that when a solution containing a biomolecule is injected into an injection target, a rate of the biomolecule that functions in the injection target is high.

### BRIEF DESCRIPTION OF THE DRAWINGS

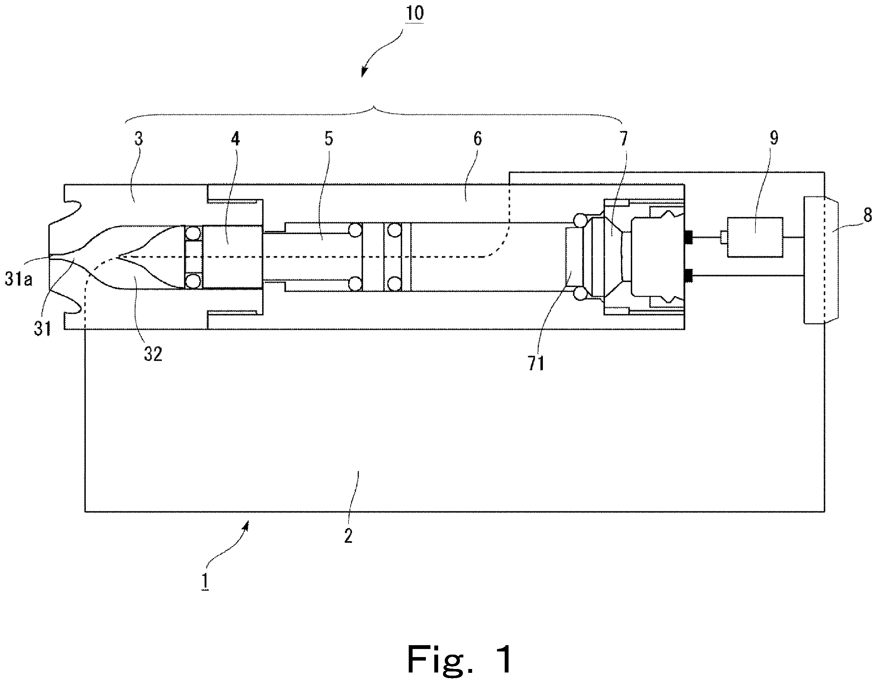

FIG. 1 is a diagram illustrating a schematic configuration of an injector according to an aspect of the present disclosure.

### MODE FOR CARRYING OUT THE INVENTION

Note that each of the configurations, combinations thereof, and the like in each of the embodiments are an example, and various additions, omissions, substitutions, and other changes may be made as appropriate without departing from the spirit of the present disclosure. The present disclosure is not limited by the embodiments and is limited only by the claims.

One aspect of the present disclosure is
an injector configured to inject a solution containing a biomolecule and a gas that is predetermined into an injection target without using an injection needle, the injector including
a storage portion configured to store the solution containing the biomolecule and the gas,
a nozzle portion communicating with the storage portion, the nozzle portion including an ejection port configured to eject the solution containing the biomolecule and the gas toward the injection target, and
a pressurization portion configured to pressurize the solution containing the biomolecule and the gas that are stored in the storage portion during an operation and to eject the solution containing the biomolecule and the gas from the ejection port toward the injection target.

### (Biomolecule)

The biomolecule to be injected into the injection target in this aspect is not particularly limited as long as the biomolecule functions in the injection target when the biomolecule is injected into the injection target. In addition, the biomolecule may be a natural product or an artificially synthesized product. Examples thereof include: a nucleic acid or a derivative thereof; a nucleoside, a nucleotide, or a derivative thereof; an amino acid, a peptide, a protein, or a derivative thereof; a lipid or a derivative thereof; a metal ion; a low molecular weight compound or a derivative thereof; an antibiotic; a vitamin or a derivative thereof. As long as the biomolecule is a nucleic acid, the biomolecule may be DNA or RNA, which may contain a gene. In Examples, which will be described below, free plasmid DNA containing a luciferase gene is used as a biomolecule, and the luciferase gene is used as a reporter gene.

The biomolecule to be injected into the injection target may be in a free form or in a form fixed to a carrier such as a nanoparticle, or in a modified form, and is not particularly limited, including a solvent, as long as when the biomolecule is injected into the injection target, the biomolecule functions in the injection target, is stably present, and has no adverse effect such as destruction of the injection target.

When the DNA contains a gene, it is designed that the gene is contained in an expression cassette or an expression vector, or the like. Furthermore, for example, the gene may be placed under control of a promoter suitable for the injection target and the injection area into which the DNA is to be injected. That is, known genetic engineering techniques can be used in any of the aspects. In the examples, which will be described below, a mammalian expression vector pGL4.13[luc2/SV40] Vector (available from Promega Corporation) is used as the expression vector. The plasmid vector is known and available to those skilled in the art. Subcloning of the expression vector and a recombinant vector can be performed according to a known method.

### (Gas That Is Predetermined)

In the injector according to this aspect, the storage portion contains a gas that is predetermined.

The gas is not particularly limited as long as when the solution containing the biomolecule is injected into the injection target, the biomolecule functions in the injection target, the biomolecule is stably present, and there is no adverse effect such as destruction of the injection target, and air can be exemplified as the gas. The air may be commonly used air, the composition of which is not particularly limited. Examples of the air include a mixed gas containing about 80% of nitrogen and about 20% of oxygen. Further, other examples of air can include nitrogen, oxygen, ozone, carbon dioxide, hydrogen, and carbon monoxide, as well as a mixed gas of any two or more of these. In addition, in a preferred aspect, the gas is a gas that does not include microorganisms or the like, or a gas in which even in a case where microorganisms are included, the microorganisms are dead.

In this aspect, it is presumed that the presence of the gas in the storage portion causes the gas to be dissolved in the solution containing the biomolecule during pressurization and starts the injection of the solution containing the biomolecule into the injection target, and the gas dissolved in the solution containing the biomolecule partially returns to a gas during depressurization. Thus, the solution containing the biomolecule can be injected into the injection target while a large shear stress is being applied to the injection target, thereby increasing a rate of the biomolecule functioning in the injection target. In addition, when the solution containing the biomolecule is injected into a cell, an amount of the solution containing the biomolecule injected into the cell through the cell membrane is preferably increased by such a mechanism, and as a result, it is presumed that the rate of the biomolecule functioning in the injection target is increased.

In this case, when a volume of the gas stored in the storage portion is too large with respect to a capacity of the storage portion, the amount of the solution containing the biomolecule in the storage portion is small. Thus, it is presumed that the injection of the solution containing the biomolecule into the injection target is completed before all of the gas is dissolved in the solution containing the biomolecule, and a sufficient shear stress is not applied to the injection target. That is, even when the volume of the gas stored in the storage portion is large with respect to the capacity of the storage portion, it is presumed that the rate of the biomolecule that functions in the injection target does not necessarily increase.

On the other hand, when the volume of the gas stored in the storage portion is too small with respect to the capacity of the storage portion, same as the above, the gas is dissolved into the solution containing the biomolecule at the same time as the pressurization and injection into the injection target of the solution containing the biomolecule is started, and a part of the gas dissolved into the solution containing the biomolecule is returned to a gas at the time of depressurization, but it is presumed that a shear stress applied to the injection target is small because the amount of the gas dissolved into the solution containing the biomolecule is small. As a result, it is presumed that the rate of the biomolecule functioning in the injection target is reduced.

From these viewpoints, the volume of the gas stored in the storage portion is preferably equal to or greater than 20%, and more preferably equal to or greater than 30%, and on the other hand, is preferably equal to or less than 60%, and more preferably equal to or less than 50% with respect to the capacity of the storage portion.

### (Function of Biomolecule)

As the examples of the case where the rate of the biomolecule functioning in the injection target is high, the following case where the DNA as the biomolecule contains a gene may be noted.

In a case where an expression amount of the gene per an amount of DNA per unit injected into the injection target when the solution containing the DNA is injected into the injection target without containing the gas in the storage portion (that is, when the storage portion is filled with the solution containing the biomolecule) (this is referred to as an aspect C) is referred to as an "expression amount C", and
an expression amount of the gene per an amount of DNA per unit injected into the injection target when the storage portion stores the solution containing the DNA and the gas in a manner that the total volume of the solution containing the DNA and the gas is the same as the volume of the solution containing the DNA contained in the storage portion in the aspect C and the solution is injected into the injection target is referred to as an "expression amount A",
the expression amount C < the expression amount A is satisfied.

As the confirmation method, for example, as in examples, which will be described later, a tissue is removed in a cylindrical shape having a freely selected radius around an injection port after the injection, a sample is prepared by a known biological method, and the confirmation can be performed by using an expression amount assay of the gene. A known method can be appropriately used depending on a type of the gene and the like. For example, when the gene is a luciferase gene, an amount of luminescence is assayed by using luciferin as a substrate.

### (Injection Target)

The injection target in the present aspect may be, for example, one or more selected from the group consisting of a cell, a cell sheet, a cell mass, a tissue, an organ (skin, a body part, or the like), an organ system, an individual (living body), and the like, and is not limited. Any of an in vitro system, an in vivo system, and an ex vivo system may be adoptable. The cell mass may be a cell mass obtained by three dimensional culture, and the organ (the skin, the body part, or the like) may be an organoid.

In addition, when injection is performed to the injection target, the injection may be performed to a lower layer included in the injection target. That is, for example, when an individual (living body) is an injection target, injection may be performed to a tissue included in the individual (living body), may be performed to a cell included in the individual (living body), or may be performed to both of the tissue and the cell. In addition, when a tissue is an injection target, injection may be performed to a cell included in the tissue, may be performed to an intercellular matrix included in the tissue, or may be performed to both of the cell and the intercellular matrix.

In addition, when the injection target in the present aspect is one or more selected from the group consisting of a cell, a cell sheet, a cell mass, a tissue, an organ (skin, a body part, or the like) and an organ system, the injection target may be one or more selected from the group consisting of a cell, a cell sheet, a cell mass, a tissue, an organ (skin, a body part, or the like), and an organ system existing in an individual (living body), or one or more selected from the group consisting of a cell, a cell sheet, a cell mass, a tissue, an organ (skin, a body part, or the like), and an organ system not existing in an individual (living body) (for example, in a state extracted or separated from an individual (living body) or a state produced outside an individual (living body)).

Additionally, the injection target in the present aspect may be one or more selected from the group consisting of a cell, a cell sheet, a cell mass, a tissue, an organ (skin, a body part, and the like), and an organ system derived from a stem cell such as an iPS cell (an induced pluripotent stem cell), and may be present in an individual (living body) or not present in an individual (living body) (for example, in a state extracted or separated from an individual (living body), or a state produced outside an individual (living body)). The cell mass may be a cell mass obtained by three dimensional culture, and the organ (the skin, the body part, or the like) may be an organoid.

The individual (living body) is preferably a mammalian individual (living body). The mammalian individual is not particularly limited, but examples of the mammalian individual include humans and mammals other than the humans. Examples of the mammals other than the humans include mice, rats, guinea pigs, hamsters, cows, goats, sheep, pigs, monkeys, dogs, and cats.

In addition, regardless of what the injection target of the present aspect is among the examples described above, when injection is performed to a cell, the injection may be performed to cytoplasm of the cell, the injection may be performed to the cell nucleus of the cell, or the injection may be performed to both the cytoplasm and the cell nucleus of the cell.

In addition, the injection target of the present aspect is not particularly limited but is preferably one or more selected from the group consisting of an intradermal part, a subcutaneous part, and a muscle layer positioned underneath the skin of an individual (living body) of a mammal. In this case, a method may be employed in which the solution containing the biomolecule and the gas are ejected from the injector toward the surface of the skin and then are injected into the skin, and the solution is injected into one or more selected from the group consisting of an intradermal part, a subcutaneous part, and a muscle layer positioned underneath the skin.

### (Injector)

In the injector of the present aspect, the term "distal end side" means a side on which the ejection port through which the solution containing the biomolecule and the gas that is predetermined are ejected from the injector is disposed, and the term "base end side" means a side opposite to the distal end side in the injector, and these terms do not limitedly indicate a specific portion or position.

In the injector of the present aspect, the drive portion applies ejection energy, and thus, the solution containing the biomolecule and the gas are ejected toward the injection target. The "ejection" by the injector according to the present aspect is achieved by using the ejection energy by the drive portion, pressurizing the solution containing the biomolecule and the gas that are stored in the storage portion by the pressurization portion, and causing the solution containing the biomolecule and the gas to flow through a flow path of the storage portion. The ejection energy may be ejection energy to be used in a typical injector, and for example, combustion energy of an explosive or the like, generation energy of a gas generating agent or the like, electric energy of a piezoelectric element or the like, or mechanical energy of a spring or the like may be used, or energy obtained by appropriately combining these forms of energy may be used.

In a case where the combustion energy of the explosive is used as the ejection energy, it is preferable to use an explosive, for example, any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive composed of a plurality of these explosives in combination. As characteristics of these explosives, the combustion product is gas at a high temperature but does not include a gas component at a room temperature, and hence, the combustion product is condensed immediately after the ignition. Thus, during a pressurization process for ejection of the solution containing the biomolecule and the gas, the temperature of the combustion product under the pressurization can be shifted by the combustion of an ignition agent to around the normal temperature in a short period of time after the pressure applied to the solution containing the biomolecule and the gas reaches a first peak injection force.

Further, when the generation energy by the gas generating agent is used as the ejection energy, the gas generating agent may be a single base smokeless explosive (for example, a single base smokeless explosive containing 98 mass% of nitrocellulose, 0.8 mass% of diphenylamine, and 1.2 mass% of potassium sulphate), or any of various gas generating agents used in gas generators for air bags and gas generators for seat belt pretensioners.

In the injector of the present aspect, the pressurization portion pressurizes the solution containing the biomolecule and the gas that are stored in the storage portion during the operation, which causes the solution containing the biomolecule and the gas to be ejected from the ejection port toward the injection target.

The pressurization by the pressurization portion is not particularly limited as long as a system is not destroyed, for example, like the destruction of the storage portion, and pressurization conditions of an ordinary injector may be employed.

In this context, the pressure means a pressure in the storage portion. While a method for measuring the pressure is not particularly limited, for example, when an injector described in the examples, which will be described later, is used for the measurement, the method in the "Method for Measuring Pressure in Storage Portion" section, which will be described later, can be used for the measurement.

The ejection energy by the drive portion is transmitted to a plunger through a piston, and the plunger slides in the storage portion, whereby the solution containing the biomolecule and the gas that are stored in the storage portion are pushed out along the flow path formed in the nozzle portion, and finally ejected from the ejection port toward the injection target.

The solution containing the biomolecule and the gas may be stored or does not need to be stored in the storage portion from the beginning, and in the case where the solution containing the biomolecule and the gas are not stored in the storage portion, the solution containing the biomolecule and the gas can be stored in the storage portion by being sucked into the storage portion through a nozzle including an ejection port. Employing the configuration that requires the storing operation into the storage portion in this manner allows a solution containing a biomolecule and a gas that are required and freely selected to be injected into the injection target. For this reason, in the injector according to the present aspect, a syringe portion and an injector body are detachably configured.

In addition, the storage portion stores the solution containing the biomolecule and the gas, and it is preferable that the solution containing the biomolecule be stored on the distal end side in the storage portion. In other words, it is preferable that the gas be stored on the base end side in the storage portion. This also means that it is preferable that the gas should not be stored on the distal end side in the storage portion.

For example, the solution containing the biomolecule can be stored on the distal end side in the storage portion and prepared by first sucking the solution containing the biomolecule into the storage portion through the nozzle including the ejection port, then sucking the gas into the storage portion, and then lightly shaking the injector or the like. Alternatively, the solution containing the biomolecule can be prepared by first sucking the gas into the storage portion through the nozzle including the ejection port, and then sucking the solution containing the biomolecule into the storage portion while maintaining the position of the gas in a manner that the stored gas is present on the base end side in the storage portion.

The injector according to the present embodiment can be obtained by storing the solution containing the biomolecule and the gas in the storage portion in a typical injector that can be used to inject an injection solution into an injection target without using an injection needle. Examples of such typical injectors include, the injector described in WO 2019/156238 A1, the injector described in WO 2019/156239 A1, the injector described in WO 2019/156237 A1, and the injector described in JP 5989039 B2.

With reference to the drawings, an injector 1 (needleless injector) will be described below as an example of the injector according to the present embodiment. Note that a configuration according to the following embodiment is provided as an example, and the disclosure is not limited to the configuration according to the present embodiment. Note that as terms indicating a relative positional relationship in a longitudinal direction of the injector 1, the term "distal end side" and the term "base end side" are used. The "distal end side" represents the position which is deviated toward the distal end of the injector 1 as described later on, i.e., deviated toward the ejection port 31a. The "base end side" represents the direction opposite to the "distal end side" in the longitudinal direction of the injector 1, i.e., the direction toward the drive portion 7 side. In addition, the present example is an example in which combustion energy of an explosive ignited by an ignition device is used as ejection energy for pressurization, but the present aspect is not limited to this.

### Configuration of Injector 1

FIG. 1 is a cross-sectional view of the injector 1, taken along the longitudinal direction thereof, illustrating a schematic configuration of the injector 1. The injector 1 is formed by attaching an injector assembly 10 to a housing (injector housing) 2. The injector assembly 10 includes a subassembly including a syringe portion 3 and a plunger 4 and a subassembly including an injector body 6, a piston 5, and a drive portion 7, and the subassemblies are integrally assembled.

As described above, the injector assembly 10 is configured to be attachable and detachable to and from the housing 2. A storage portion 32 formed between the syringe portion 3 and the plunger 4 that are included in the injector assembly 10 is filled with a solution containing a biomolecule and a gas, and the injector assembly 10 is a unit that is thrown away each time the solution containing the biomolecule and the gas are ejected. On the other hand, a battery 9 that supplies power to an igniter 71 included in the drive portion 7 of the injector assembly 10 is included on the housing 2 side. The power supply from the battery 9 is performed between an electrode on the housing 2 side and an electrode on the drive portion 7 side of the injector assembly 10 through wiring lines, when a user performs an operation of pressing a button 8 provided to the housing 2. Note that the electrode on the housing 2 side and the electrode on the drive portion 7 side of the injector assembly 10 have shapes and positions designed to come into contact with each other automatically when the injector assembly 10 is attached to the housing 2. Further, the housing 2 is a unit that can be repeatedly used as long as power that can be applied to the drive portion 7 is left in the battery 9. Note that when the battery 9 runs out of power in the housing 2, the housing 2 may be continued to be used with only the battery 9 exchanged.

Next, the details of the injector assembly 10 will be described. First of all, description is given on the subassembly including the syringe portion 3 and the plunger 4. Inside the syringe portion 3, the storage portion 32 is formed as a space in which the solution containing the biomolecule and the gas can be stored. More specifically, as illustrated in FIG. 1, the plunger 4 is disposed to be slidable along an inner wall surface extending in the axial direction of the syringe portion 3, and the storage portion 32 is defined by the inner wall surface of the syringe portion 3 and the plunger 4. Additionally, the syringe portion 3 includes a nozzle portion 31 communicating with the storage portion 32, and the nozzle portion 31 is formed with the ejection port 31a on the distal end side. The nozzle portion 31 is a flow path whose flow path cross-sectional area gradually decreases from the storage portion 32 side toward the ejection port 31a side, and the flow path is configured to guide the solution containing the biomolecule and the gas that is predetermined that are filled in the storage portion 32 to the ejection port 31a. In the example illustrated in FIG. 1, the plunger 4 has a shape on the distal end side that substantially matches a shape of the nozzle portion 31.

Next, the subassembly including the injector body 6, the piston 5, and the drive portion 7 will be described. The piston 5 is made of metal, for example, and is configured to be pressurized by a combustion product (combustion gas) generated by the igniter 71 of the drive portion 7 and to slide in a through hole formed in the injector body 6. The injector body 6 is a substantially cylindrical member, and the piston 5 is contained therein to be slidable along the inner wall surface extending in the axial direction thereof. Note that the piston 5 may be formed of resin, and in such a case, metal may be used together for a part to which heat resistance and pressure resistance are required. Additionally, as illustrated in FIG. 1, the piston 5 is integrally coupled with the plunger 4.

Next, the drive portion 7 will be described. As illustrated in FIG. 1, the drive portion 7 is fixed to a base end side with respect to the through hole in the injector body 6. The drive portion 7 includes the igniter 71, which is an electric igniter. The igniter 71 is disposed to face the interior of the through hole in the injector body 6 and contains an ignition agent therein. As the ignition agent, various types of explosives can be employed as described above. In addition, the ignition agent can be contained in an explosive cup formed by an appropriate thin metal, for example.

In the injector 1 configured as described above, a volume of the gas stored in the storage portion 32 is adjusted to be, for example, equal to or greater than 20% and equal to or less than 60% of a capacity of the storage portion 32.

Next, how the injector 1 having the configuration described above is operated will be described. As illustrated in FIG. 1, after the injector assembly 10 is attached to the housing 2, the solution containing the biomolecule and the gas that is predetermined are sucked from the ejection port 31a of the nozzle portion 31. This allows the solution containing the biomolecule and the gas that is predetermined to be filled in the storage portion 32. In this state, for example, with the ejection port 31a being in contact with the injection target in the injector 1, when a user performs an operation of pressing the button 8 provided to the housing 2, this serves as a trigger and actuation power is supplied from the battery 9 to the igniter 71 of the drive portion 7, and thus, the igniter 71 is activated. When the igniter 71 is activated, the ignition agent is ignited and thus combusted, and combustion products (flame, combustion gas, and the like) are generated. As a result, an explosive cup of the igniter 71 is ruptured, for example, and the combustion gas of the ignition agent is released into the through hole in the injector body 6. Thus, the pressure in the through hole of the injector body 6 suddenly increases, and the piston 5 is pressed toward the distal end side of the injector body 6. As a result, the piston 5 slides along the inner wall surface of the through hole in the injector body 6 toward the distal end side. As described above, because the plunger 4 is coupled integrally with the piston 5, the plunger 4 also slides along the inner wall surface of the syringe portion 3 in conjunction with the piston 5. That is, with the plunger 4 pushed toward the nozzle portion 31 positioned on the distal end side of the syringe portion 3, the capacity of the storage portion 32 storing the solution containing the biomolecule and the gas that is predetermined decreases, and rapid pressurization occurs. As a result, the solution containing the biomolecule and the gas that is predetermined that are filled in the storage portion 32 are pushed into the nozzle portion 31 and are ejected from the ejection port 31a at a high pressure. Thus, the solution containing the biomolecule and the gas that is predetermined can be injected into the injection target.

Further, although an additional explosive component is not particularly disposed in the injector body 6 illustrated in FIG. 1, a gas generating agent or the like, which is combusted by a combustion product generated by the explosive combustion at the igniter 71 and generates gas, may be disposed in the igniter 71 or the through hole of the injector body 6, and thus, a change in pressure applied to the solution containing the biomolecule and the gas through the piston 5 is adjusted. The configuration in which the gas generating agent is disposed in the igniter 71 is a known technique as disclosed in WO 01/31282 A1, JP 2003 25950 A, and the like. Additionally, as one example of the gas generating agent, there may be exemplified a single base smokeless explosive containing 98 mass% of nitrocellulose, 0.8 mass% of diphenylamine, and 1.2 mass% of potassium sulphate. Further, various types of gas generating agents used in a gas generator for an air bag and a gas generator for a seat belt pretensioner may be used. A combustion completion time period of the gas generating agent can be changed by adjusting dimensions, a size, a shape, and particularly, a surface shape of the gas generating agent in being disposed in the through hole, which allows a change in pressure to be applied to the solution containing the biomolecule and the gas to be adjusted in a desired change, that is, in a change that causes the solution containing the biomolecule and the gas to appropriately reach the injection target. In the present aspect, the gas generating agent to be used as necessary is also included in the drive portion 7. In the present embodiment, the plunger 4 and the piston 5 constitute the "pressurization portion".

Another aspect of the present disclosure is a method of injecting a solution containing a biomolecule and a gas that is predetermined into an injection target by using the injector of the above aspect.

Regarding the injector, the injection target, and the solution containing the biomolecule in this aspect, the description in the above-described aspect is applied thereto.

### EXAMPLES

Examples will be described below, but none of the examples shall be construed as limiting the present disclosure.

### (Example 1)

pGL4.13[luc2/SV40] Vector (available from Promega Corporation) was prepared as a plasmid, and a required amount of the plasmid was amplified to adjust its concentration to 1 µg/µL.

The plasmid solution was sucked into the storage portion (capacity: 100 µL) of the injector at 100 µL, 80 µL, 70 µL, 50 µL, 40 µL, or 30 µL from a nozzle of the injector. Thereafter, except for the case where 100 µL of the plasmid solution was sucked up, the plunger was pulled up to a scale of 100 µL without sucking up the plasmid solution, and the air in the ordinary laboratory was filled. That is, a volume of the air stored in the storage portion was respectively 0%, 20%, 30%, 50%, 60%, and 70% of a capacity of the storage portion (hereinafter, the volume of the air stored in the storage portion with respect to the capacity of the storage portion may be referred to as an "air abundance rate"), and an amount of the plasmid to be injected was respectively 100 µg, 80 µg, 70 µg, 50 µg, 40 µg, and 30 µg. In addition, the air in the storage portion during the injection was stored on the base end side, and the plasmid solution was stored on the distal end side.

Here, the injector was used as a device for injecting the above-mentioned injection substance into the abdomen of a test animal, which will be described later, and is the injector illustrated in FIG. 1. In this example, 35 mg of ZPP was used as the ignition agent, and 40 mg of a single base smokeless explosive (containing 98 mass% of nitrocellulose, 0.8 mass% of diphenylamine, and 1.2 mass% of potassium sulphate) was used as the gas generating agent.

Note that known techniques were used to measure a period of time required for a pressure to reach the maximum pressure from the start of pressurization, and the maximum pressure. That is, like the measurement method described in JP 2005 021640 A, measurement was performed by a method in which an ejection force was applied in a dispersed manner to a diaphragm of a load cell disposed downstream of a nozzle and an output from the load cell was collected by a data collection device via a detection amplifier and was stored as an ejection force (N) per unit time. The ejection pressure measured in this manner was divided by the area of the ejection port 31a of the injector, and thus, the ejection pressure was calculated. The measurement value obtained by the internal pressure measurement of the storage portion is equivalent to the ejection pressure, and the ejection pressure can be regarded as the pressure inside the storage portion. As a result, for example, when the volume of the air stored in the storage portion was 0% of the capacity of the storage portion, a period of time required for a pressure to reach the maximum pressure from the start of pressurization was 0.55 milliseconds, and the maximum pressure was 10.3 MPa.

A domestic pig (Specific Pathogen Free, SPF) was used as a test animal, anesthetized in advance and shaved, then the abdomen was injected with the injection substance inside the injector. After the injection, the animal was kept for about 24 hours and euthanized by blood removal under anesthesia. The injection area was hollowed out with a biopsy punch ϕ of 8.0 mm (available from Kai corporation), collected in a tube of 2.0 mL, added with Passive Lysis 5 × Buffer (available from Promega Corporation) diluted in 5 times with Milli-Q water 1 mL by 1 mL, and minced with scissors until skin pieces of about 1 mm were obtained. After freezing on dry ice once and melting at a normal temperature once, the skin pieces were separated by centrifugation with 20380G for 2 minutes and thus, a supernatant containing a protein extract solution was obtained. Luciferase Assay System (available from Promega) was used for luciferase measurement. That is, 20 µL of the protein extract solution was added to and mixed with 100 µL of a substrate solution, and an amount of luminescence (RLU) was measured with Lumitester C (available from Kikkoman Corporation).

Since the amount of the plasmid to be injected into the abdomen varies depending on the above-mentioned conditions, an expression efficiency was calculated as an expression amount per 1 µg of the plasmid and expressed as a relative value with respect to the case where the air abundance rate was 0%. The results are shown in Table 1.

**Table 1**

| Air abundance Rate (%) | Amount of plasmid (µg) | Mean of measurement values (A.U.) | Relative value (times) per 1 µg of plasmid |
|---|---|---|---|
| 0 | 100 | 218438.4 | 1.00 |
| 20 | 80 | 248189.8 | 1.42 |
| 30 | 70 | 1189736.4 | 7.78 |
| 50 | 50 | 323908.4 | 2.97 |
| 60 | 40 | 236994.4 | 2.71 |
| 70 | 30 | 79265.8 | 1.21 |

From these results, it was confirmed that the expression efficiency of luciferase is remarkably improved when the air abundance rate is equal to or greater than 20% and equal to or less than 60%.

### (Reference Example 1)

The following comparative experiment was separately conducted. That is, the same operation as in Example 1 was conducted except that the same amount of a TE buffer solution (available from NACALAI TESQUE, INC.) as that of the air was filled instead of the air. As a result, it was confirmed that the concentration of the plasmid does not affect the expression efficiency per 1 µg of the plasmid.

### EXPLANATION OF REFERENCES

1 injector; 2 housing; 3 syringe portion; 4 plunger; 5 piston; 6 injector body; 7 drive portion; 8 button; 9 battery; 10 injector assembly; 31 nozzle portion; 31a ejection port; 32 storage portion; 71 igniter

## Claims

1. An injector (1) configured to inject a solution containing a biomolecule and a gas that is predetermined into an injection target without using an injection needle, the injector (1) comprising:
a storage portion (32) configured to store the solution containing the biomolecule and the gas;
a nozzle portion (31) communicating with the storage portion (32), the nozzle portion (31) including an ejection port (31a) configured to eject the solution containing the biomolecule and the gas toward the injection target; and
a pressurization portion (4, 5) configured to pressurize the solution containing the biomolecule and the gas that are stored in the storage portion (32) during an operation and to eject the solution containing the biomolecule and the gas from the ejection port (31a) toward the injection target.

2. The injector (1) according to claim 1, wherein a volume of the gas stored in the storage portion (32) is equal to or greater than 20% and equal to or less than 60% of a capacity of the storage portion (32).

3. The injector (1) according to claim 1 or 2, wherein the gas is air.

4. The injector (1) according to any one of claims 1 to 3, wherein the biomolecule is DNA containing a gene.

5. A method of injecting a solution containing a biomolecule and a gas that is predetermined into an injection target by using the injector (1) according to any one of claims 1 to 4.
